# EUROPÄISCHE PATENTSCHRIFT

(11) **EP 2 108 943 B1**
(45) Veröffentlichungstag und Bekanntmachung des Hinweises auf die Patenterteilung: **29.10.2014**
(21) Anmeldenummer: 09004957.8
(22) Anmeldetag: 03.04.2009
(51) Int. Cl.: G01N 21/64, A61B 1/00, A61B 1/04, A61B 5/00, G02B 23/24, H04N 13/02, A61B 1/06, G01N 21/17, G01N 21/47

(54) **Vorrichtung und Verfahren zur Fluoreszenz-Bildgebung**
Device and method for fluorescence imaging
Dispositif et procédé d'imagerie à fluorescence

(30) Priorität: 11.04.2008 DE 102008018637
(43) Veröffentlichungstag der Anmeldung: 14.10.2009
(73) Patentinhaber: Storz Endoskop Produktions GmbH, 78532 Tuttlingen (DE)
(72) Erfinder: Krattiger, Beat, Dr., CH-8222 Beringen (CH)
(74) Vertreter: Fugmann, Winfried

(56) Entgegenhaltungen:
- EP-A1- 1 746 410
- EP-A1- 1 759 628
- DE-A1- 19 626 433
- JP-A- 2002 286 639
- US-B1- 6 825 455
- WAGNIERES G ET AL: "Frequency-domain fluorescence lifetime imaging for endoscopic clinical cancer photodetection: apparatus design and preliminary results" JOURNAL OF FLUORESCENCE 1997 MAR PLENUM PUBL CORP, Bd. 7, Nr. 1, März 1997 (1997-03), Seiten 75-83, XP002566365
- C.G. MORGAN ET AL.: "New Approaches to Lifetime-Resolved Luminescence Imaging" JOURNAL OF FLUORESCENCE, Bd. 7, Nr. 1, 1997, Seiten 65-73, XP002566366

## Beschreibung

Die vorliegende Erfindung betrifft eine Vorrichtung zur Fluoreszenz-Bildgebung nach dem Oberbegriff von Anspruch 1 .

Vorrichtungen und Verfahren zur Fluoreszenz-Bildgebung sind bekannt. So wird beispielsweise in WO97/11636 eine Vorrichtung zur Diagnose mittels einer durch einen Photosensibilisator oder durch Eigenfluoreszenz hervorgerufenen Reaktion in biologischem Gewebe beschrieben, bei der ein Beleuchtungssystem zur Erzeugung von Fluoreszenzanregungslicht vorgesehen ist sowie eine lichtzuführende Einheit zur Beleuchtung eines zu beobachtenden Gewebebereichs mit dem Fluoreszenzanregungslicht. Das von dem Gewebebereich kommende Licht kann über eine Endoskopoptik aufgenommen und in einer proximalen Bildebene abgebildet werden, in der beispielsweise der Bildaufnehmer einer Videokamera angeordnet sein kann. Durch Verwendung geeigneter Filter kann auf diese Weise ein Bild der Fluoreszenzintensität im betreffenden Gewebebereich erzeugt werden, das beispielsweise auf einem Videoschirm dargestellt werden kann.

Ein solches Fluoreszenzbild gestattet die Bestimmung der Verteilung und der Aktivität von Fluoreszenzstoffen im Gewebe. Da diese in sehr sensibler Weise vom Stoffwechsel und der Umgebung der Körperzellen abhängen, ermöglicht die Messung der Fluoreszenzstrahlung den Nachweis von Gewebeveränderungen, die durch Beobachtung des reflektierten Lichts nicht oder noch nicht erkennbar sind. Insbesondere bösartige Gewebeveränderungen oder Tumoren können auf diese Weise bereits im Frühstadium erkannt werden. Die Fluoreszenzstoffe können dabei körpereigene Stoffe (Autofluorophore) sein, die im Körpergewebe von Natur aus vorhanden sind. Es ist aber auch möglich, Fluoreszenzstoffe für die Diagnose zuzuführen, oder Stoffe zuzuführen, aus denen die Fluoreszenzstoffe entstehen. Ein solcher Photosensibilisator ist beispielsweise 5-Aminolävulinsäure (5-ALA); ein auf 5-ALA basierendes Medikament wird von der Fa. GE Healthcare unter dem Namen HEXVIX® angeboten. Durch Stoffwechselvorgänge, die in praktisch allen Körperzellen stattfinden können, entsteht aus 5-ALA das fluoreszenzfähige Protoporphyrin IX (PpIX), welches mit einer Anregungsstrahlung im Bereich von 405 nm zur Fluoreszenz im Bereich von ca. 635 nm angeregt werden kann, die bei einer Fluoreszenzdiagnose beobachtet wird. Da diese Stoffwechselvorgänge von der Art, dem Zustand und der Umgebung der Zellen abhängig sind, ermöglicht die Fluoreszenz einen Rückschluss auf den Zustand des Gewebes und die Erkennung entarteten Gewebes.

Gemäß WO97/11636 kann zur besseren Orientierung und für eine kontrastreichere Darstellung zusätzlich zur Fluoreszenzstrahlung auch ein Anteil des reflektierten Beleuchtungslichts vom Detektor aufgenommen und auf dem Videoschirm dargestellt werden. Dabei müssen die spektralen Eigenschaften der Komponenten so aufeinander augestimmt abgestimmt sein, dass das sehr viel hellere Anregungslicht die Fluoreszenz im dargestellten Bild nicht überstrahlt.

Die Beobachtung der Fluoreszenzstrahlung hat als Autofluoreszenz (AF)- bzw. photodynamische Diagnose (PDD) inzwischen eine große Bedeutung für die medizinische Diagnostik erlangt. Ein solches System zur endoskopischen Autofluoreszenz-Diagnose von Bronchialerkrankungen wird beispielsweise von der Fa. KARL STORZ angeboten (s. Firmenschrift "Autofluoreszenz-Bronchoskopie", EndoGramm Thor 1-1-D/11-2005).

In EP 1 759 628 A1 ist eine optische Abbildungsvorrichtung offenbart mit einer Lichtquelle, einem Einführungsabschnitt, der in eine Körperhöhle eingeführt werden kann, einem Lichtübertragungsabschnitt im Einführungsabschnitt, der Licht von der Lichtquelle zu einem Gegenstand in der Körperhöhle führt, einem optischen Lichtaufnahmesystem im Einführungsabschnitt, das von dem Gegenstand zurückkommendes Licht aufnimmt, einem Abschnitt zur Aufnahme eines Bilds im reflektierten Licht und einem Abschnitt zur Aufnahme eines Fluoreszenzbilds.

Die von einem Fluoreszenzstoff emittierte Fluoreszenzstrahlung unterscheidet sich von reflektierter oder gestreuter Strahlung durch die Zeitspanne, die verstreicht, bis nach der Absorption eines oder mehrerer Photonen des Anregungslichts ein oder mehrere Photonen der Fluoreszenzstrahlung emittiert werden. Diese Zeitspanne liegt für viele in biologischem Gewebe vorhandene oder durch Photosensibilisatoren erzeugte Fluoreszenzstoffe im Bereich von Nanosekunden (ns). In den letzten Jahren sind Systeme zum Nachweis der Zeitverzögerung bei der Fluoreszenzstrahlung entwickelt worden. Da die Zeitverzögerung mit der Lebensdauer des entsprechenden angeregten Zustands des Fluoreszenzstoffs zusammen hängt, spricht man hierbei von Fluoreszenzlebensdauer-Messungen. Eine ortsaufgelöste Abbildung der Zeitverzögerung bzw. Fluoreszenzlebensdauer wird allgemein als "Fluorescence Lifetime Imaging" (FLIM) bezeichnet.

Aus JP2002286639 A ist eine Vorrichtung zur zeitaufgelösten Fluoreszenzdetektion bekannt, bei der Anregungslicht abgestrahlt wird, und die ein phasenauflösendes Element in einem optischen Pfad des Anregungslichts, ein optisches System zur Erfassung von Fluoreszenz, die durch das Anregungslicht erzeugt wird, und ein Kontrollelement zur Kontrolle des Zeitverhaltens des Anregungslichts und der Fluoreszenz umfasst.

In DE 196 26 433 A1 wird ein Endoskopkopf beschrieben, der zum Anbau an einen Endoskopschlauch vorgesehen ist und der lichtemittierende Elemente, eine Endoskopoptik und eine Bilderzeugungseinrichtung aufweist. Zur Trennung verschiedener Fluoreszenzen nach ihrer Abklingzeit ist eine Hochfrequenzansteuemng einer LED in Verbindung mit einem ebenfalls angesteuerten CCD-Chip vorgesehen.

Aus EP 1746410 A1 ist eine mikroskopische Vorrichtung bekannt, bei der ein Objekt mit hochfrequent modulierter Strahlung beleuchtet und mit einem phasenempfindlichen Festkörperdetektor, der eine Mehrzahl von Pixeln umfasst, beobachtet wird. Der Detektor ermöglicht die pixelweise phasenselektive Speicherung von Ladungsträgern, die durch die auftreffende Strahlung ausgelöst werden, und das pixelweise Auslesen der gespeicherten Ladungen. Durch eine entsprechende Auswertung der verschiedenen Phasen des auftreffenden Signals zuzuordnenden Ladungswerte ist pixelweise eine Bestimmung der Phasendifferenz zwischen dem empfangenen Signal und der Beleuchtungsstrahlung möglich, die wiederum ein Maß für die Fluoreszenzlebensdauer ist. Die Fluoreszenzanregungsstrahlung wird über ein Mikroskopobjektiv in die Objektebene fokussiert, die Beobachtung der Fluoreszenzstrahlung erfolgt über dasselbe Mikroskopobjektiv.

In dem Artikel von Elson et al., Ann. Rev. in Fluorescence 2006, p. 1 - 50, wird ein endoskopisches System zur Fluoreszenz-Bildgebung beschrieben, bei dem von einer Lichtquelle eine gepulste Anregungsstrahlung ausgesandt wird, die über eine Lichtleitfaser auf einen endoskopisch zu beobachtenden Gewebebereich geleitet wird. Über das Okular einer Endoskopoptik wird die von dem Gewebebereich emittierte Fluoreszenzstrahlung auf die Photokathode eines Gated Optical Image Intensifiers (GOI) abgebildet. Die Zahl der in einem Zeitfenster mit gegenüber den Pulsen der Anregungsstrahlung vorbestimmbarer Verzögerung gezählten Ladungsträger, die von der auftreffenden Fluoreszenzstrahlung erzeugt werden, steht pixelweise zur Erzeugung eines Fluoreszenzlebensdauerbilds zur Verfügung. Um ein Auseinanderlaufen der Pulse zu verhindern, wird ein diodengepumpter Nd:YVO₄-Laser verwendet, der eine sehr geringe Divergenz aufweist und dessen Energie in die tiefen Moden einer Lichtleitfaser mit besonders niedriger Gruppengeschwindigkeitsdispersion eingekoppelt wird. Ein GOI erfordert eine stabile mechanische Halterung, aufwändige Elektronik, hohe Spannungen und ist für die Handhabung an einem Endoskop bei einer endoskopischen Operation nicht geeignet.

In dem Artikel von Wagnières et al., Frequency-domain Fluorescence Lifetime Imaging for Endoscopic Clinical Cancer Photodetection: Apparatus Design and Preliminary Results, in: Journal of fluorescence, Vo. 7, No. 1, 1997, S. 75 - 83, wird ein Versuchsaufbau beschrieben, bei dem kontinuierlich moduliertes Fluoreszenzanregungslicht auf einen endoskopisch zu beobachtenden Gewebebereich geleitet wird. Die von dem Gewebebereich ausgehende Strahlung wird über eine Endoskopoptik auf zwei Bildverstärker mit moduliertem Verstärkungsfaktor geleitet. Das von diesen Bildverstärkern erzeugte stationäre Bild wird von jeweils einer CCD-Kamera aufgenommen und zur Erzeugung eines Fluoreszenzlebensdauerbilds ausgewertet. Dieser Aufbau erfordert einen sehr hohen instrumentellen Aufwand und ist deshalb für den routinemäßigen klinischen Einsatz nicht geeignet.

Es ist daher die Aufgabe der vorliegenden Erfindung, eine Vorrichtung zur Fluoreszenz-Bildgebung anzugeben, die zur endoskopischen Anwendung, beispielsweise auch intraoperativ, geeignet ist, und kostengünstig und leicht zu handhaben ist. Ebenso ist es die Aufgabe der vorliegenden Erfindung, ein entsprechendes Verfahren zur Fluoreszenz-Bildgebung anzugeben. Unter "Fluoreszenz" werden hier und im Folgenden auch andere Formen der Lumineszenz, insbesondere auch Phosphoreszenz, verstanden.

Diese Aufgabe wird durch eine Vorrichtung gemäß Anspruch 1 gelöst.

Dadurch, dass die Fluoreszenzanregungsstrahlung kontinuierlich modulierbar ist, dass der erste Bildsensor ein Festkörperdetektor ist, der phasenempfindlich ansteuerbar ist, und dass die von dem ersten Bildsensor gelieferten Daten pixelweise Phaseninformationen der Fluoreszenzstrahlung enthalten, wird erfingdungsgemäß eine endoskopisch anwendbare Vorrichtung zur Fluoreszenz-Bildgebung geschaffen, die kostengünstig und einfach zu handhaben ist. Eine kontinuierliche Modulation der Fluoreszenzanregungsstrahlung kann mit relativ einfachen elektronischen Mitteln erzeugt werden, und phasenempfindliche Festkörpersensoren sind einfach aufgebaut, leicht zu handhaben und kostengünstig verfügbar. Mit einer entsprechenden Modulationsfrequenz können Zeitverzögerungen, die im Bereich der Lebensdauern häufige Fluoreszenzstoffe liegen, einfach und sicher nachgewiesen werden. Durch die pixelaufgelöste Erfassung und Auswertung der Phaseninformationen ist die Erzeugung eines Bildes, das ortsaufgelöste Fluoreszenzlebensdauerinformationen darstellt, möglich. Hierdurch kann FLIM beispielsweise für viele diagnostische Anwendungen in der klinischen Praxis verfügbar gemacht werden.

Eine erfindungsgemäße Vorrichtung umfasst Lichterzeugungsmittel zur Erzeugung mindestens einer modulierten Fluoreszenzanregungsstrahlung. Zur Erzeugung der Fluoreszenzanregungsstrahlung können dabei insbesondere lichtemittierende Dioden (LEDs), Superlumineszenzdioden, Laser, insbesondere Laserdioden, oder andere entsprechend modulierbare Strahlungsquellen eingesetzt werden. Sofern hierfür eine Lichtquelle mit intrinsisch pulsartigem Output, beispielsweise ein Superkontinuum-Laser, verwendet wird, müssen die erzeugten Lichtpulse bevorzugt in eine kontinuierliche bzw. kontinuierlich modulierte Strahlung umgesetzt werden, beispielsweise mittels eines optischen Pulsverlängerers (Pulse Stretcher). Hierdurch kann das Ausgangssignal, das bei Superkontinuum-Weißlichtlasern intrinsisch typischerweise weniger als ca. 10% einer Periode andauert, auf über 50% oder nahe 100% verlängert werden, wobei eine ausreichende Modulation zur Anwendung des erfindungsgemäßen Verfahrens erhalten bleibt. Ein solcher Pulse Stretcher kann hier in vorteilhafter Weise dadurch realisiert werden, dass die Anregungsstrahlung in eine Multimode-Glasfaser, insbesondere eine Multimode-Stufenindex-Glasfaser, mit einer ausreichend hohen numerischen Apertur (NA) eingekoppelt wird und durch die Weglängenunterschiede von Strahlen mit unterschiedlichem Einkopplungswinkel über eine ausreichende Faserlänge entsprechende Zeitverzögerungen und damit eine Pulsverbreiterung bzw. Pulsverlängerung entstehen. So reicht beispielsweise eine NA von ca. 0.6 aus, um bei einer Glasfaserlänge von ca. 40 m die erforderliche Pulsverlängerung in der Größenordnung von 20 ns zu erzielen.

Hierbei haben insbesondere Laserdioden den Vorteil einer einfachen Handhabung und sind kostengünstig, kompakt und leicht modulierbar. Dabei haben multimodale Laserdioden i. d. R. den Vorteil einer höheren Ausgangsleistung als monomodale Laserdioden. Erfindungsgemäß können Laserdioden mit einer intrinsischen Divergenz weit über 1mrad, beispielsweise 0,1 rad, bis über 0,9 rad verwendet werden.

Die Fluoreszenzanregungsstrahlung ist dabei kontinuierlich modulierbar, d. h., über die gesamte oder zumindest einen wesentlichen Teil einer Modulationsperiode wesentlich von Null verschieden und/oder zeitlich veränderlich. Insbesondere ist die Fluoreszenzanregungsstrahlung kontinuierlich periodisch modulierbar. Anders als bei einer pulsartigen Modulation, die durch steile Flanken gekennzeichnet ist und dadurch, dass diese nur einen geringen Teil, beispielsweise 10%, insbesondere weniger als 50% einer Periode ausfüllt, soll hier die Anregungsstrahlung während eines großen Teils der Periode andauern, und auch die zeitliche Ableitung des Modulationssignals soll bevorzugt in der Größenordnung derjenigen bei einer sinusförmigen Modulation liegen. Insbesondere kann die Intensität der Fluoreszenzanregungsstrahlung sinusförmig oder quasi-sinusförmig moduliert sein, ggf. über einem Grundniveau.

Die erzeugte Strahlung enthält, bei Erzeugung oder nach nachträglicher Umwandlung, mindestens Licht einer solchen Wellenlänge, die zur Anregung mindestens eines Fluoreszenzstoffes oder Fluorophors in einem zu beobachtenden Bereich geeignet ist. Unter "Licht" wird hier nicht nur sichtbares Licht, sondern beispielsweise auch infrarote oder ultraviolette Strahlung verstanden. Zur besseren Handhabung und effektiveren Kühlung können die Lichterzeugungsmittel in einem eigenen Gehäuse bzw. als separate Lichtquelle angeordnet sein.

Weiterhin umfasst eine erfindungsgemäße Vorrichtung Lichtweiterleitungsmittel zur Weiterleitung der Fluoreszenzanregungsstrahlung auf einen zu beobachtenden Bereich. Die Lichtweiterleitungsmittel können dabei insbesondere Mittel zur Einkopplung der von der Lichtquelle erzeugten Strahlung in einen Lichtleiter umfassen, sowie Lichtleiter zur Weiterleitung der Strahlung. So kann beispielsweise eine Linsen- und/oder Spiegelanordnung zur verbesserten Einkopplung der erzeugten Strahlung vorgesehen sein, oder es können auch fasergekoppelte Superlumineszenz- oder Laserdioden verwendet werden. Erfindungsgemäß kann die Einkopplung mit einer numerischen Apertur bis über 0,25 rad erfolgen, was die Verwendung einer Vielzahl gängiger Superlumineszenz- oder Laserdioden ermöglicht. Sind die Lichterzeugungsmittel in einem eigenen Gehäuse bzw. als separate Lichtquelle angeordnet, kann insbesondere ein Lichtleitkabel zur Weiterleitung der Strahlung vorgesehen sein, das mit Verbindungsmitteln zur Verbindung mit der Lichtquelle oder weiteren Lichtleitern versehen sein kann.

Ferner ist wenigstens eine endoskopisch einsetzbare Beleuchtungsoptik vorgesehen. Eine solche Beleuchtungsoptik kann flexibel, halbstarr oder starr sein und aus einer Lichtleitfaser, einem Bündel von Lichtleitfasern, einem Lichtleitstab oder einer anderen Einrichtung zur Lichtweiterleitung bestehen, die in einen Hohlraum eines Gegenstands oder eines menschlichen oder tierischen Körpers hineinbringbar ist. Die Lichtleitfasern können insbesondere Multimode-Fasern sein. Es können auch Mittel zur Verteilung der Strahlung auf den zu beobachtenden Bereich vorgesehen sein, wie beispielsweise ein Diffusor oder eine Aufweitungsoptik zur gleichmäßigen Ausleuchtung des Objektfelds, insbesondere am distalen (objektnahen) Ende der Beleuchtungsoptik. Insbesondere dann, wenn die Strahlung mit einer hohen numerischen Apertur eingekoppelt bzw. weitergeleitet wird, kann die Aufweitungsoptik ganz oder teilweise entfallen.

Zur Vermeidung der Einkopplung unerwünschter Strahlung, beispielsweise zur Verringerung der Wärmebelastung bei einer endoskopischen Anwendung in einem lebenden Körper, können weiterhin Filtermittel vorgesehen sein, die bestimmte Anteile der erzeugten Strahlung ganz oder teilweise wegfiltern. Solche Filtermittel können insbesondere auch vorteilhaft sein, um zu verhindern, dass unerwünschte Strahlung von einem Bildsensor aufgenommen wird und dadurch dessen Funktion oder die Aussagekraft des von diesem erzeugten Bilds beeinträchtigt.

Die erfindungsgemäße Vorrichtung umfasst weiterhin Lichtabbildungsmittel zur Abbildung einer Fluoreszenzstrahlung aus dem zu beobachtenden Bereich mindestens auf einen ersten Bildsensor, wobei die Lichtabbildungsmittel mindestens eine endoskopisch einsetzbare, flexible, halbstarre oder starre Abbildungsoptik umfassen. Hierfür kann insbesondere eine Linsenanordnung, beispielsweise ein Endoskopobjektiv, vorgesehen sein. Dieses kann eine Abbildung auf einen nahe dem distalen Ende der Abbildungsoptik angeordneten Bildsensor erzeugen. Es können aber auch Bildweiterleitungsmittel vorgesehen sein, beispielsweise ein Stablinsensystem oder ein Bildleiter aus Lichtleitfasern, um das von dem Endoskopobjektiv erzeugte Bild an das proximale (objektferne) Ende der Abbildungsoptik weiterzuleiten, wo die Abbildung auf einen Bildsensor vermittelt wird. Die Lichtabbildungsmittel können weiterhin Filtermittel umfassen, um bestimmte Anteile der aufgenommenen Strahlung abzublocken, beispielsweise die Fluoreszenzanregungsstrahlung, die das Fluoreszenzsignal überstrahlen kann.

Ferner umfasst die erfindungsgemäße Vorrichtung Steuerungsmittel zur Steuerung der Lichterzeugungsmittel und zur Ansteuerung des ersten Bildsensors, der als ein phasenempfindlich ansteuerbarer Festkörpersensor ausgebildet ist. Insbesondere ermöglichen die Steuerungsmittel die Erzeugung der Modulation der Fluoreszenzanregungsstrahlung und eine entsprechende Ansteuerung des ersten Bildsensors zur phasenaufgelösten Aufnahme der empfangenen Strahlung und zum Auslesen des Signals des ersten Bildsensors.

Schließlich sind erfindungsgemäß Auswertungsmittel vorgesehen zur Auswertung der von dem ersten Bildsensor, der eine Mehrzahl von Pixeln aufweist, gelieferten Daten, die pixelweise Phaseninformationen in Bezug auf die Modulation der Fluoreszenzstrahlung enthalten, zur Erzeugung eines Fluoreszenzbilds. Dabei kann aus den Phaseninformationen auf die Zeitverzögerung zwischen Auftreffen der Fluoreszenzanregungsstrahlung und Aussendung der Fluoreszenzstrahlung geschlossen werden, um ein Bild zu erzeugen, das beispielsweise pixelweise diese Zeitverzögerung, die mit der Fluoreszenzlebensdauer zusammenhängt, darstellt. Beispielsweise durch eine Vergleichsmessung mit nicht-fluoreszierendem Material können Entfernungs- und apparative Effekte von der Zeitverzögerung durch Fluoreszenz unterschieden werden, so dass die Fluoreszenzlebensdauer bestimmt werden kann. Ein solches Bild kann schließlich beispielsweise auf einem Videoschirm dargestellt werden.

Hierdurch wird die erfindungsgemäße Aufgabe vollständig gelöst.

Erfindungsgemäß sind die Beleuchtungsoptik und die Abbildungsoptik in einem gemeinsamen endoskopisch einsetzbaren Schaft angeordnet. Dieser Schaft kann, je nach Ausbildung der Beleuchtungs- und der Abbildungsoptik, ebenfalls flexibel, halbstarr oder starr ausgebildet sein. Dies hat den Vorteil, dass die erfindungsgemäße Vorrichtung bei endoskopischen Anwendungen einfach zu bedienen ist und der Schaft in bekannter Weise durch eine natürliche oder künstliche Körperöffnung in einen körperinneren Hohlraum oder auch beispielsweise durch eine Inspektionsöffnung in das Innere eines technischen Bauteils eingerührt werden kann. Dabei kann es aus Gründen der Handhabung vorteilhaft sein, wenn die Lichterzeugungsmittel als kompakte Einheit mit dem Schaft verbunden sind. Hierfür sind insbesondere Lichterzeugungsmittel mit geringer Verlustleistung und Baugröße geeignet, beispielsweise Superlumineszenz- oder Laserdioden.

Erfindungsgemäß ist mindestens der erste Bildsensor in einer Kameraanordnung aufgenommen, die mit dem endoskopisch einsetzbaren Schaft lösbar verbunden ist. Insbesondere kann die Kameraanordnung eine kompakte Kameraeinheit mit einem Gehäuse umfassen, das beispielsweise über eine Endoskop-Kupplung einfach und sicher mit dem Endoskopschaft verbindbar ist.

Es wurde festgestellt, dass bei einer kontinuierlichen, insbesondere sinusförmigen Modulation der Fluoreszenzanregungsstrahlung eine etwaige Gruppengeschwindigkeitsdispersion in Materialien, die zur Lichtweiterleitung verwendet werden, sich allenfalls auf die Signalamplitude, jedoch nicht wesentlich auf die gemessene Zeitverzögerung bzw. Fluoreszenzlebensdauer auswirkt. Erfindungsgemäß können deshalb beispielsweise für die Weiterleitung der Fluoreszenzanregungsstrahlung Materialien mit einer nicht verschwindenden Gruppengeschwindigkeitsdispersion und/oder Multimodefasern verwendet werden. Multimodefasern sind zudem für die Übertragung von Licht unvollständiger Kohärenz günstiger.

Gemäß einer weiteren bevorzugten Ausführungsform können die Beleuchtungsoptik, die Abbildungsoptik und der gemeinsame endoskopisch einsetzbare Schaft deshalb Teile eines Standard-Endoskops sein, das ein starres, halbstarres oder flexibles Endoskop sein kann. Ein solches Standard-Endoskop kann ein Okular für einen direkten Einblick aufweisen oder auch für den Anschluss einer Kameraeinheit mittels eines C-Mount vorbereitet sein.

Dies hat den Vorteil, dass vorhandene, beispielsweise in einer Klinik verfügbare Endoskope erfindungsgemäß für die Fluoreszenz-Bildgebung verwendet werden können. Solche Endoskope stehen in einer Vielzahl von Ausführungen für verschiedene human- und tiermedizinische oder technische Anwendungen zur Verfügung. Es können aber auch im distalen Bereich weitere, fest oder lösbar damit verbundene Komponenten vorgesehen sein, wie beispielsweise Strahlteiler, Bildsensor usw., die für die Fluoreszenz-Bildgebung spezifisch sind. In diesem Fall ermöglicht die Verwendung der übrigen Komponenten eines Standard-Endoskops zumindest eine kostengünstige Herstellung, insbesondere durch Verwendung von Materialien, deren Biokompatibilität, Sterilisierbarkeit, Hitzebeständigkeit usw. erwiesen sind.

In einer nicht beanspruchten Ausgestaltung sind zumindest die Abbildungsoptik und der erste Bildsensor innerhalb eines endoskopisch einsetzbaren Schafts nahe dessen distalem Ende angeordnet. Ferner kann auch die Beleuchtungsoptik nahe dem distalen Ende, sowie weitere optische und elektronische Bauelemente innerhalb des Schafts audgenommen sein. Dies hat den Vorteil, dass lediglich elektrische und/oder Lichtleitkabel die Verbindung zwischen distalem und proximalem Ende des Schafts herstellen müssen, was eine besonders einfache, kostengünstige und schlanke Ausführung des Schafts ermöglicht, insbesondere eines flexiblen Schafts. In einer bevorzugten Weiterbildung der Erfindung sind zumindest die Abbildungsoptik und der erste Bildsensor, vorteilhafterweise auch weitere optische und elektronische Bauelemente, zu einer in Art eines Wechselobjektivs vom verbleibenden Teil des Schafts und des Endoskops trennbaren Einheit zusammengefasst. Dies hat den weiteren Vorteil, dass eine Nutzung des Endoskops auch für andere Anwendungsfälle als die Fluoreszenz-Bildgebung möglich ist.

Für die Erzeugung der Fluoreszenzanregungsstrahlung können Leuchtmittel unterschiedlicher Kohärenz verwendet werden. Bei Beleuchtung mit Strahlung hoher Kohärenz kann das entstehende Bild durch Speckles beeinträchtigt sein. Diese erzeugen scheinbare Strukturen in der Beleuchtungsstärke und/oder im beobachteten Signal, die die Strukturen des beobachteten Objekts überdecken können. Gemäß einer bevorzugten Ausführungsform der Erfindung sind deshalb Mittel zur Verringerung der Kohärenz vorgesehen, um ein gleichmäßiges Bild zu erzeugen. So können beispielsweise die Leuchtmittel derart angesteuert werden, dass der Kohärenzgrad unterhalb einer vorgegebenen Schwelle bleibt oder dann, wenn im erzeugten Fluoreszenzbild in erheblichem Maße Speckles auftreten, der Kohärenzgrad reduziert wird. Dies kann beispielsweise bei einer Laserdiode durch Betrieb an bzw. dicht unter der Laserschwelle erreicht werden, es kann aber auch die Laserdiode derart angesteuert bzw. moduliert werden, dass die Kohärenz der emittierten Strahlung geringer ist.

Bei einer erfindungsgemäßen Vorrichtung zur Fluoreszenz-Bildgebung kann auch ein Okular vorgesehen sein. Der direkte Einblick durch ein Okular hat den Vorteil, dass auch schon unabhängig von der Bilderzeugung durch den phasenempfindlichen Bildsensor eine Beobachtung des zu beobachtenden Gebiets möglich ist, um beispielsweise einem Arzt in der gewohnten Weise eine einfache Orientierung in einem Körperhohlraum bzw. im dem zu beobachtenden Gewebebereich zu ermöglichen.

Erfindungsgemäß sind zusätzlich zur dem phasenempfindlichen Bildsensor ein oder mehrere weitere Sensoren vorhanden. Dies können Sensoren zur nicht-bildgebenden Analyse der Strahlung sein, wobei aber erfindungsgemäße mindestens ein weiterer Bildsensor vorhanden ist. Solche Bildsensoren können beispielsweise als CCD- oder als andere Halbleiter-Bildsensoren ausgebildet sein, insbesondere als UV-, IR-, Restlicht- oder weitere phasenempfindliche Bildsensoren.

Die Verwendung weiterer Sensoren ermöglicht die Gewinnung zusätzlicher Informationen aus dem beobachteten Bereich, wie etwa spektraler oder Intensitätsinformationen. Die Verwendung weiterer Bildsensoren hat den besonderen Vorteil, dass als Ergänzung zum Fluoreszenzlebensdauerbild mindestens ein weiteres Bild zur Verfügung steht. Dadurch ist beispielsweise bei einer diagnostischen Anwendung die Erkennung von Strukturen möglich, die allein im Fluoreszenzlebensdauerbild nicht oder nicht deutlich genug hervortreten, oder die Korrelation der im Fluoreszenzlebensdauerbild erkennbaren Strukturen mit Strukturen, die mit anderen Bildgebungsmethoden erkennbar sind. Ebenso kann ein dem visuellen Eindruck entsprechendes Bild, beispielsweise ein mit einem oder mehreren Sensoren aufgenommenes RGB-Bild, oder ein Bild in einem eingeschränkten Spektralbereich aufgenommen werden.

Erfindungsgemäß ist ein Strahlteiler vorgesehen. Dieser dient zur Aufteilung der empfangenen Strahlung auf verschiedene Beobachtungsstrahlengänge bzw. Sensoren. Als Strahlteiler können insbesondere ein oder mehrere halbdurchlässige Spiegel, bevorzugt in Form teilverspiegelter Flächen in Bildteilerprismen oder Bildteilerwürfeln, verwendet werden, wobei die halbdurchlässigen Spiegel auch als dichroitische Spiegel zur spektral selektiven Bildaufteilung ausgebildet sein können. Die Verwendung halbdurchlässiger Spiegel hat den Vorteil, dass zu jedem Zeitpunkt eine Informationsgewinnung über verschiedene Beobachtungsstrahlengänge möglich ist.

Ergänzend hierzu können auch Mittel zum Umschalten zwischen verschiedenen Beobachtungsstrahlengängen bzw. Sensoren vorgesehen sein. Dies können mechanische Mittel sein, beispielsweise ein Chopper, oder auch elektrische Mittel, wie ein elektrisch ansteuerbarer Spiegel. Die Umschaltmittel können auch mit den Lichterzeugungsmitteln und/oder mit dem phasenempfindlichen Bildsensor synchron ansteuerbar sein. Das Umschalten ermöglicht in vorteilhafter Weise die abwechselnde Nutzung der verschiedenen Beobachtungsstrahlengänge.

Gemäß einer weiteren bevorzugten Ausführungsform der Erfindung ist eine Anpassungsoptik vorgesehen zur Anpassung der Gesichtsfelder des ersten und mindestens eines weiteren Bildsensors. Wenn aus prinzipiellen Gründen oder auch aus Platz- oder Kostengründen Bildsensoren verwendet werden, die unterschiedliche Formate aufweisen, hat dies den Vorteil, das ggf. unterschiedliche Sensorgrößen bzw. Bilddiagonalen und/oder Sensorformate nicht zu unterschiedlich großen Gesichtsfeldern führen, sondern die verschiedenen Bilddaten jeweils das gleiche Gebiet repräsentieren. Dies ist insbesondere vorteilhaft, wenn ein einfacher visueller Vergleich der verschiedenen Bilddaten durch einen Anwender ermöglicht werden soll, und dieser nicht auf das jeweils kleinste Bildfeld der unterschiedlichen Sensoren beschränkt sein soll.

Die Anpassungsoptik ist zwischen Strahlteiler und mindestens einem Bildsensor angeordnet und kann dabei aus einem optischen Glied oder mehreren bestehen und kann insbesondere Linsen mit negativer und/oder positiver Brechkraft enthalten oder entsprechende Spiegelelemente. Hierdurch kann das auf einem Bildsensor erzeugte Bild vergrößert oder verkleinert werden, so dass es etwa das gleiche Gesichtsfeld abbildet wie auf einem oder mehreren anderen Bildsensoren. Zur Anpassung an unterschiedliche Formate bzw. Seitenverhältnisse der Bildsensoren kann die Anpassungsoptik auch astigmatische optische Elemente enthalten. Die Anpassungsoptik kann auch zusammen mit einem Bildteilerprisma oder -würfel als kompakter Block ausgebildet sein. Eine Vergrößerungs-Anpassungsoptik kann auch eine Verkleinerung des Strahlteilers und damit eine Gewichts-, Volumen- und Kostenreduktion ermöglichen.

Zur Einstellung, Fokussierung und/oder Justage kann die Anpassungsoptik auch veränderlich, beispielsweise als Zoomoptik, ausgebildet sein. Dies ist insbesondere dann vorteilhaft, wenn der erste und/oder der mindestens eine weitere Bildsensor vom Strahlteiler bzw. einer optischen Einheit, die den Strahlteiler umfasst, trennbar ausgebildet sind. In diesem Fall kann beim Ansetzen eines Bildsensors eine neue Einstellung der Bildebene und/oder Vergrößerung erforderlich sein, Insbesondere dann, wenn ein Bildsensor durch einen andersartigen ersetzt wird, kann durch eine Anpassungsoptik die Anpassung der jeweiligen Gesichtsfelder ermöglicht werden.

Erfindungsgemäß ist ein Bildsensor vorgesehen, der zur Erzeugung eines Bilds des beobachteten Gebiets mittels des reflektierten bzw. gestreuten Lichts ausgebildet ist. Ein solches Bild hat den Vorteil, dass es beispielsweise bei einer diagnostischen Anwendung den gewohnten Anblick des zu diagnostizierenden Gewebebereichs wiedergibt und dadurch die Orientierung im Fluoreszenzlebensdauerbild und die Identifikation des beobachteten Gewebebereichs erleichtert. Zur Unterscheidung zwischen reflektiertem und gestreutem Licht können auch polarisationsoptische Elemente vorgesehen sein.

Gemäß einer weiteren bevorzugten Ausführungsform der Erfindung kann ein spektral aufgelöstes bzw. selektiertes Bild des beobachteten Bereichs erzeugt werden. Hiefür können beispielsweise Filter vorgesehen sein, die nur einen bestimmten spektralen Anteil des reflektierten bzw. gestreuten Lichts hindurch lassen. Ein spektral in mehrere Anteile aufgelöstes bzw. nach einem spektralen Anteil selektiertes

Bild erlaubt eine kontrastreichere Darstellung und verbessert dadurch die Sicherheit einer Diagnose.

Insbesondere für den Fall einer schmalbandigen spektral selektiven Bildgebung kann ein spektral selektives Element, wie ein Prisma oder ein Gitter vorgesehen sein. Ein Abbild des beobachteten Bereichs entsteht dann durch Punkt- oder Linienscannen. Hierdurch kann ein besonders kontrastreiches Bild erzeugt werden.

Gemäß einer weiteren bevorzugten Ausführungsform ist ein Bildensor zur Erzeugung eines Fluoreszenz-Intensitätsbilds vorgesehen. Der weitere Bildsensor kann insbesondere in an sich bekannter Weise als Bildsensor für ein PDD-System ausgebildet sein. Das erzeugte PDD-Bild kann dabei auch einen Anteil des reflektierten bzw. gestreuten Lichts enthalten. Das Fluoreszenz-Intensitätsbild steht als ergänzend Darstellung der gleichen oder anderer Fluoreszenzstoffe, die im Fluoreszenzlebensdauerbild beobachtet werden, zur Verfügung. Hierdurch ist eine verbesserte Diagnose möglich, insbesondere kann dadurch die Spezifität der Diagnose erhöht werden und beispielsweise falsch positive Befunde und damit etwa unnötige Biopsien vermieden werden.

Erfindungsgemäß ist es vorgesehen, dass zusätzlich zu einem Fluoreszenzlebensdauerbild auch 3D-Daten des beobachteten Gebiets erzeugt werden. Dies hat den Vorteil, dass zusätzliche Informationen über das beobachtete Gebiet gewonnen werden können, die zur Identifikation beispielsweise eines Gewebebereichs und zur Diagnose dienen können.

Erfindungsgemäß werden die 3D-Daten mittels desselben phasenempfindlichen Bildsensors und in besonders bevorzugter Weise mit der gleichen Beleuchtung und Datenauswertung gewonnen. Hierfür ist lediglich eine andere Filterung notwendig, die auch das reflektierte bzw. gestreute Licht durchlässt. Während für das Fluoreszenzbild das reflektierte bzw. gestreute Fluoreszenzanregungslicht störend ist und deshalb vorteilhafterweise eliminiert wird, wird dieses bei der Gewinnung von Entfernungs- bzw. 3D-Daten verwendet, wobei das Fluoreszenzlicht eliminiert werden kann. Es kann auch beispielsweise durch ein Kantenfilter, dass nur für einen Wellenlängenbereich durchlässig ist, der praktisch keine Fluoreszenz anregt, aber noch im Detektionsbereich des Sensors liegt, etwa im roten oder NIR-Bereich, beispielsweise ab 800 nm, sichergestellt werden, dass nur für die 3D-Datengewinnung verwendbares Licht auf das Objekt eingestrahlt wird und als reflektiertes bzw. gestreutes Licht zum Sensor gelangt.

Da die 3D-Daten direkte Informationen über die Zeitverzögerung zwischen Beleuchtung und reflektiertem bzw. gestreutem Signal enthalten, kann hiermit auch derjenige Anteil der Zeitverzögerung zwischen Beleuchtung und Fluoreszenzsignal, der nicht auf die Fluoreszenzlebensdauer, sondern auf die Entfernung der fluoreszierenden Oberfläche zurückzuführen ist, korrigiert werden. Hierdurch kann eine genauere Darstellung der Fluoreszenzlebensdauer erhalten werden.

Gemäß einer weiteren bevorzugten Ausführungsform sind die 3D-Daten zur Flächen- und/oder Volumenmessung auswertbar. Aus einem Fluoreszenzlebensdauerbild oder aus einem anderen Bild des be-obachteten Bereichs kann unter Zuhilfenahme der Entfernungsinformationen die laterale Ausdehnung einer Struktur ermittelt werden, sowie aus den Entfernungsdaten die Tiefenausdehnung der Struktur. Auf diese Weise ist die Messung von Flächen und Volumina möglich. Dies ist insbesondere bei endoskopischen Anwendungen vorteilhaft, wo eine Tiefeninformation nicht ohne weiteres vorhanden ist. Sowohl bei der Inspektion technischer Bauteile als auch bei der medizinischen Diagnose von Gewebeveränderungen sind solche Messungen wünschenswert zur Bestimmung der Größe und, bei mehreren Messungen, zeitlichen Veränderung von Strukturen wie Rissen in technischen Bauteilen oder Läsionen oder Tumoren in menschlichen oder tierischen Organen. In besonders vorteilhafter Weise kann die Messung von Flächen oder Volumina kombiniert werden mit Fluoreszenzdaten aus dem Fluoreszenzlebensdauerbild oder auch aus einem Fluoreszenzintensitätsbild.

In einer weiteren bevorzugten Ausführungsform der Erfindung wird aus den 3D-Daten ein Stereobild synthetisiert. Ein solches Stereobild ist mit einer geeigneten Anzeigeeinrichtung, die einem Betrachter für beide Augen jeweils unterschiedliche Bilder anzeigt, darstellbar und vermittelt dem Betrachter einen wirklichkeitsnahen räumlichen Eindruck vom beobachteten Gebiet. Dies ist insbesondere wünschenswert bei endoskopischen Operationen zur Verbesserung der intuitiven Kontrolle des Operateurs über die von ihm bedienten Instrumente.

Die erfindungsgemäße Vorrichtung kann gemäß einer besonders bevorzugten Ausführungsform derart ausgebildet sein, dass die von dem ersten und mindestens einem weiteren Bildsensor gelieferten Bilddaten oder auch von einer am Okular angeschlossenen Kamera gelieferten Bilddaten in einer synoptischen Weise dargestellt werden können. Eine solche Darstellung kann eine überlagerte Darstellung der betreffenden Bilddaten sein, eine abwechselnde bzw. eine Darstellung nacheinander oder eine Darstellung in nebeneinander angeordneten Bildern. Für die Darstellung für einen Benutzer können entsprechende Anzeigeeinrichtungen vorgesehen sein, wie beispielsweise ein oder mehrere Videoschirme, Projektoren, Drucker usw.. Ebenso können Speichermittel zur Aufzeichnung, Dokumentation und Archivierung der Bilddaten vorgesehen sein. Die Steuerungs- und Auswertemittel können ferner zur Durchführung von Bildanalyseverfahren, die vom Benutzer unabhängig oder von diesem einstellbar sein können, ausgebildet sein. Hierdurch können beispielsweise Kontraste hervorgehoben oder verringert werden, die Daten geglättet oder auch Strukturen aus den von verschiedenen Bildsensoren gelieferten Bilddaten miteinander korreliert werden. So können beispielsweise Informationen aus einem visuellen bzw. RGB-Bild, aus AF- bzw. PDD-Bildern und/oder einem Fluoreszenzlebensdauerbild miteinander kombiniert werden.

Die Steuerungs- und Auswerteeinrichtung kann auch dazu ausgebildet sein, durch Korrelation der Bilddaten der verschiedenen Bildsensoren automatisch eine lagerichtige Überlagerung der Bilddaten herzustellen oder auch eine automatische Anpassung der Gesichtsfelder bezüglich Lage, Größe und/oder Format durchzuführen. Ebenso kann, ggf. sensorabhängig, eine automatische Fokussierung oder eine automatische Verzeichnungskorrektur vorgesehen sein.

Gemäß einer weiteren bevorzugten Ausführungsform der Erfindung kann es vorgesehen sein, nicht nur solches Licht zu erzeugen und zu dem zu beobachtenden Gebiet weiterzuleiten, das zur Anregung mindestens eines Fluoreszenzstoffs geeignet ist, sondern noch mindestens eine weitere Strahlung. Diese kann beispielsweise weißes Licht sein zur Erzeugung eines Weißlichtbildes bei Beobachtung durch ein Okular oder mittels eines entsprechenden weiteren Bildsensors oder auch mittels des ersten Bildsensors in einem entsprechenden Betriebsmodus. Hierfür können entsprechende Lichterzeugungsmittel vorhanden sein, beispielsweise eine Xenon- oder Metall-Halid-Lampe zur Erzeugung einer Weißlichtbeleuchtung, die in einer eigenen Lichtquelle oder zusammen mit den Lichterzeugungsmitteln für die Fluoreszenzanregungsstrahlung in einer gemeinsamen Lichtquelle angeordnet sein können.

Ebenso können für die Erzeugung einer breitbandigen Beleuchtung auch fasergepumpte Fluoreszenzlichtquellen, wie beispielsweise in US 20070092184 A1 offenbart, verwendet werden, wenn diese genügend kurze Fluoreszenzlebensdauern aufweisen. Bei der Verwendung von Superkontinuum-Weißlichtlaserquellen ergibt sich der zusätzliche Vorteil, dass diese, ggf. synchron mit dem phasenempfindlichen Bildsensor, modulierbar sind. Darüber hinaus können Superkontinuum-Weißlichtlaser wellenlängenselektiv strahlen, und sind entsprechend elektrisch ansteuerbar. Eine solche Lichtquelle kann zur Erzeugung aller benötigten Strahlungsarten ausreichen.

Häufig kann für die Erzeugung eines Bilds im reflektierten bzw. gestreuten Licht die Fluoreszenzanregungsstrahlung selbst verwendet werden; dabei kann es vorgesehen sein, die Fluoreszenzstrahlung durch ein geeignetes Filter herauszufiltern, in der Regel wird jedoch die Fluoreszenzstrahlung durch die reflektierte bzw. gestreute Anregungsstrahlung überstrahlt werden, so dass ein entsprechendes Filter nicht notwendig ist. In vorteilhafter Weise kann für die Erzeugung eines solchen Bildes aber auch eine breitbandige, insbesondere eine Weißlichtbeleuchtung, beispielsweise durch eine Xenon-Lichtquelle, verwendet werden.

Die weitere Strahlung kann gleichzeitig mit der Fluoreszenzanregungsstrahlung erzeugt und in das zu beobachtende Gebiet weitergeleitet werden oder auch durch entsprechende Ansteuerung und/oder durch ein optisches Schaltelement wechselweise mit der Fluoreszenzanregungsstrahlung erzeugt bzw. weitergeleitet werden. Letzteres ist insbesondere dann vorteilhaft, wenn nicht zu allen Zeiten eine Beleuchtung mit den jeweiligen Strahlungen notwendig ist, und in den anderen Zeiten eine Abschaltung bzw. Abblockung der jeweils nicht benötigten Strahlung erfolgen kann, um die eingekoppelte Energiemenge zu reduzieren. Die Umschaltfrequenz kann so hoch gewählt werden, dass die Umschaltung mit dem Auge oder auf der Anzeigeeinrichtung nicht mehr oder nur noch wenig bemerkbar ist. Insbesondere kann die Wechselfrequenz so mit der Bildfrequenz synchronisiert werden, dass eine ganze Anzahl Videobilder auf eine halbe Wechselperiode fällt.

Ein nicht beanspruchtes Verfahren zur Fluoreszenz-Bildgebung umfasst die folgenden Schritte:
- Erzeugung mindestens einer kontinuierlich modulierten Fluoreszenzanregungsstrahlung,
- Weiterleitung der Fluoreszenzanregungsstrahlung auf einen zu beobachtenden Bereich durch mindestens eine endoskopisch einsetzbaren Beleuchtungsoptik,
- Abbildung einer Fluoreszenzstrahlung aus dem zu beobachtenden Bereich mindestens auf einen ersten Bildsensor, der als Festkörperdetektor ausgebildet ist, der phasenempfindlich angesteuert wird, über mindestens eine endoskopisch einsetzbare Abbildungsoptik, und
- Erzeugung eines Fluoreszenzbilds durch Auswertung der Phaseninformationen der Fluoreszenzstrahlung in den von dem ersten Bildsensor gelieferten Daten.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
- Fig. 1: ein bevorzugtes Ausführungsbeispiel einer erfindungsgemäßen Vorrichtung;
- Fig. 2: eine alternative Ausführungsform eines in einer erfindungsgemäßen Vorrichtung verwendeten Strahlteilers;
- Fig. 3: eine erfindungsgemäße Lichtquelle;
- Fig. 4: eine nicht beanspruchte Ausführungsform einer Vorrichtung zur Fluoreszenz-Bildgebung;
- Fig. 5: ein vergrößertes Detail aus Fig. 4.

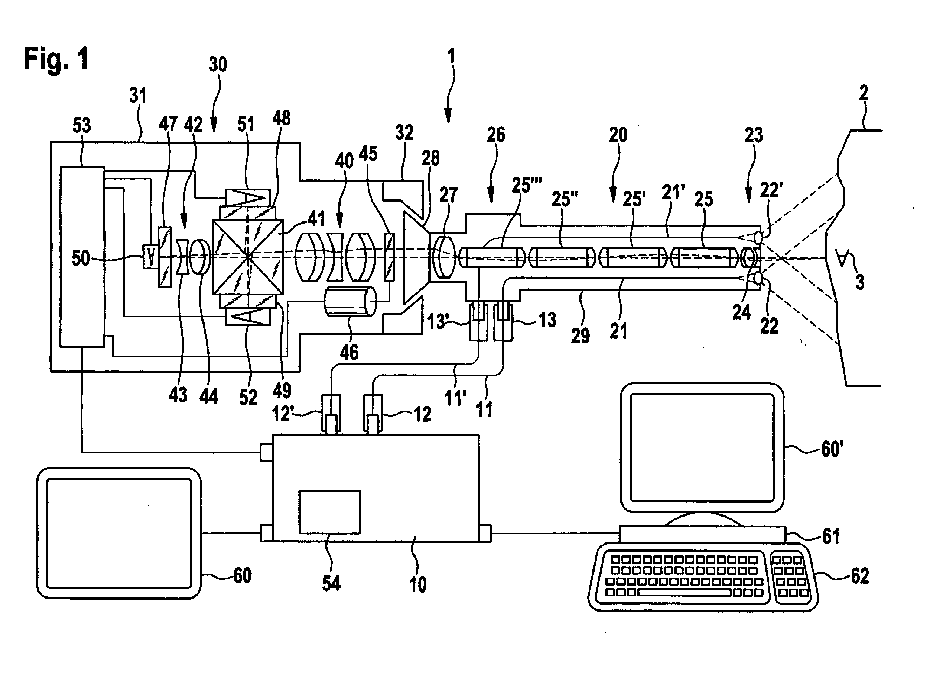

Gemäß Fig. 1 umfasst eine erfindungsgemäße Vorrichtung 1 eine Lichtquelle 10. In dem dargestellten bevorzugten Ausführungsbeispiel ist die Lichtquelle zur Erzeugung einer kontinuierlich, beispielsweise sinusförmig, modulierten Fluoreszenzanregungsstrahlung und einer Weißlichtbeleuchtung ausgebildet. Zur Weiterleitung beider Strahlungsarten sind jeweils Lichtleitkabel 11, 11' vorgesehen, die über Anschlüsse 12, 12' mit der Lichtquelle 10 und über Anschlüsse 13, 13' mit dem Endoskop 20 verbunden werden können. Bei einer entsprechenden Einkopplung oder beispielsweise bei Verwendung eines Superkontinuum-Lasers können beide Strahlungsarten auch über einen einheitlichen Lichtleiter weitergeleitet werden. Die Lichtquelle kann auch in das Endoskop integriert sein.

Über die Endoskop-Lichtleiter 21, 21' werden die Fluoreszenzanregungsstrahlung und das Weißlicht zum objektseitigen (beobachterfernen, distalen) Ende 23 des Endoskops 20 geführt. Die dort angeordneten Aufweitungsoptiken 22, 22' dienen der gleichmäßigen Verteilung der Beleuchtungsstrahlung auf das zu beobachtende Gebiet, beispielsweise ein Objekt 2, etwa ein Gewebebereich in einem körperinneren Hohlraum (nicht dargestellt).

Zur Erzeugung eines Bilds eines Teilbereichs des Objekts 2 umfasst das Endoskop 20 ein Endoskopobjektiv 24. Das von diesem erzeugte Zwischenbild wird von einem Relaislinsensystem, hier einer Anordnung von Stablinsen 25, 25', 25", 25''', an das objektferne (beobachtemahe, proximale) Ende 26 des Endoskops 20 geführt. Wird statt des dargestellten starren Endoskops 20 ein flexibles Endoskop verwendet, so kann das erzeugte Bild über Glasfaser-Bildleiter übertragen werden. Am proximalen Ende des Endoskops ist eine Okularlinsenanordnung 27 vorgesehen. Solange die Kameraanordnung 30 nicht an das Endoskop 20 angesetzt ist, ist ein direkter Einblick durch die Okularmuschel 28 möglich. Endoskop-Lichtleiter 21, 21', Endoskopobjektiv 24 und Relaislinsen 25, 25', 25'', 25'''sind dabei in einem in eine Körperöffnung einführbaren Endoskopschaft 29 aufgenommen.

Zur Fluoreszenz-Bildgebung kann an das Endoskop 20 die Kameraanordnung 30 angesetzt werden. Diese enthält in einem Gehäuse 31 einen oder mehrere optische Sensoren, sowie weitere optische und ggf. elektronische Bauelemente. Zur lösbaren Verbindung mit dem Endoskop 20 ist eine Endoskopkupplung 32 vorgesehen, die beispielsweise einen Schnapp- oder Bajonettmechanismus enthält, um eine einfache, sichere und lösbare Verbindung zu gewährleisten. Insbesondere kann eine Anschlagfläche (nicht dargestellt) vorgesehen sein, um die relative Position von Endoskop und Kameraanordnung zu fixieren, und ein Eintrittsfenster (nicht dargestellt) zur Verhinderung der Verschmutzung der Kameraanordnung.

Die Kameraanordnung 30 enthält ein Kameraobjektiv 40, einen als Teilerwürfel 41 ausgebildeten Strahlteiler zur Aufteilung des Bilds auf mehrere Bildsensoren, Bildsensoren 50, 51, 52 und eine Anpassungsoptik 42, hier exemplarisch bei dem Bildsensor 50 dargestellt. Die Anpassungsoptik 42 enthält beispielsweise eine Linse bzw. Linsengruppe mit negativer Brechkraft 43 und eine Linse bzw. Linsengruppe mit positiver Brechkraft 44 und ist so ausgebildet, dass auf die aktive Fläche des Bildsensors 50 trotz unterschiedlicher Größe das gleiche Bildfeld abgebildet wird wie auf die Bildsensoren 51 und 52. Dies ist in Fig. 1 symbolisch durch die abgebildete Struktur 3 dargestellt, die durch die als Verkleinerungsoptik ausgebildete Anpassungsoptik auf den kleineren Bildsensor 50 kleiner als auf die Bildsensoren 51 und 52 abgebildet wird.

Ferner enthält die Kameraanordnung Aufbereitungsoptiken 45, 47, 48, 49, die insbesondere als Filter und/oder als optische Schalter ausgebildet sein können. Die Aufbereitungsoptiken können beispielsweise Farbfilter, insbesondere Absorptions- oder Interferenzfilter, elektronisch abstimmbare Filter, Prismen, Verlängerungsplatten oder Raumfrequenzfilter (Anti-Aliasing Filter) umfassen. So kann insbesondere die Aufbereitungsoptik 45, die allen Bildsensoren vorgeschaltet ist, bestimmt sein, Lichtanteile, die für alle Bildsensoren unerwünscht sind, zu eliminieren. Der Aufbereitungsoptik 45 kann ein Aktuator 46, beispielsweise zum Ein- oder Ausschwenken eines Filters, zugeordnet sein. Die Aufbereitungsoptiken 47, 48, 49 dienen der insbesondere der Filterung des auftreffenden Lichts entsprechend der jeweiligen Art des Sensors.

So kann beispielsweise der Bildsensor 50 als phasenempfindlicher Festkörper-Bildsensor ausgebildet sein, der pixelweise die Intensität der auftreffenden Fluoreszenzstrahlung und die Phasenverschiebung, d. h., die Zeitverzögerung zwischen Fluoreszenzanregungsstrahlung und auftreffender Fluoreszenzstrahlung registriert. Aus den von diesem Bildsensor gelieferten Daten können ein Fluoreszenzlebensdauerbild, ein Fluoreszenzintensitätsbild und/oder 3D-Daten rekonstruiert werden.

Damit ein Fluoreszenzlebensdauerbild entsteht, ist es vorteilhaft, die zumeist viel hellere reflektierte bzw. gestreute Fluoreszenzanregungsstrahlung abzublocken. Hierfür ist die Aufbereitungsoptik 47 beispielsweise als Bandpass oder auch als Kantenfilter ausgebildet, der für die kurzwelligere Fluoreszenzanregungsstrahlung undurchlässig ist, die langwelligere Fluoreszenzstrahlung jedoch passieren lässt. Durch eine entsprechende Auswertung kann, wie beispielsweise in EP 1 746 410 A1 angegeben, pixelweise die Phasenverschiebung und damit die Zeitverzögerung ermittelt werden. Ein Fluoreszenzintensitätsbild kann mit dem gleichen Bildsensor und dem gleichen Filter, jedoch mit der Auswertung der Demodulation erzeugt werden. Ist nur eine Fluoreszenz-Bildgebung beabsichtigt, so kann das entsprechende Filter auch an einem anderen Ort in der Kameraanordnung 30 oder auch im Abbildungsstrahlengang im Endoskop 20 angeordnet sein.

Mit dem gleichen Bildsensor und der gleichen Auswertung wie für das Fluoreszenzlebensdauerbild können auch 3D-Daten erzeugt werden, insbesondere pixelweise Abstandsinformationen. Hierfür wird eine ebenfalls kontinuierlich modulierte Beleuchtungsstrahlung verwendet, die auch die Fluoreszenzanregungsstrahlung sein kann. Um alleine die durch die Entfernung zum Objekt verursachte Zeitverzögerung, nicht die durch Fluoreszenz erzeugte Zeitverzögerung, zu messen, ist es vorteilhaft, eine Beleuchtungsstrahlung zu wählen, die keine wesentliche Fluoreszenz erzeugt, also beispielsweise eine Strahlung im roten oder NIR-Bereich. Es ist aber auch möglich, die Fluoreszenzstrahlung durch ein entsprechendes Filter abzublocken. Sofern die Fluoreszenzstrahlung wesentlich schwächer als die reflektierte bzw. gestreute Strahlung ist oder außerhalb des Detektionsbereichs des phasenempfindlichen Bildsensors liegt, kann auch darauf verzichtet werden. Zur Verbesserung des Signal-Rauschverhältnisses kann der größte Teil einer Weißlichtbeleuchtung durch ein Bandpass-Filter, das für das modulierte Analyselicht durchlässig ist, geblockt werden. Beide Filter, für die Fluoreszenz- und für die Abstandsmessung, können auch umschaltbar als Aufbereitungsoptik 45 oder 47 angeordnet sein.

Der Bildsensor 51 erzeugt im Ausführungsbeispiel ein RGB-Farbbild. Die Aufbereitungsoptik 48 kann hierfür beispielsweise als IR-Sperrfilter ausgebildet sein. Der Bildsensor 52 kann beispielsweise ein Bild im NIR-Bereich erzeugen, könnte aber auch beispielsweise ein Wärmebild aufnehmen oder einen Bildverstärker zur Erhöhung der Empfindlichkeit enthalten.

Die Aufbereitungsoptiken können auch als elektronisch kontrollierbare Filter ausgebildet sein, beispielsweise als durchstimmbarer Flüssigkristallfilter (Liquid Crystal Tunable Filter, LCTF) oder als akustooptischer durchstimmbarer Filter (Acousto-Optical Tunable Filter, AOTF). Der Strahlteiler kann auch, beispielsweise durch eine dichroitische Beschichtung, spektral selektiv ausgebildet sein, so dass ein geringerer Anteil des Lichts in Sperrfiltern verloren geht.

Die Bildsensoren 50, 51, 52 sind mit einer Steuerungselektronik 53 verbunden, die die Bildsensoren kontrolliert und die Bilddaten ausliest und, ggf. nach einer ersten Vorverarbeitung, weiterleitet. Die Steuerungselektronik 53 ist auch mit der Lichtquelle 10 verbunden, so dass das Auslesen der Daten und die Modulation der Fluoreszenzanregungsstrahlung synchronisiert werden können. Ferner ist ein Steuergerät 54 vorgesehen, das beispielsweise in die Lichtquelle 10 integriert sein kann. Weiterhin sind Anzeigeeinrichtungen 60, 60' vorgesehen zur Anzeige der Bilddaten, sowie eine Eingabeeinrichtung, beispielsweise eine Tastatur 62, ein Touch Screen oder auch eine Spracherkennungseinrichtung.

Das Steuergerät 54 verarbeitet die Bildsignale für eine unmittelbare Anzeige, steuert die Lichtquelle 10 synchron mit den Bildsensoren, steuert ggf. ansteuerbare Filter und leitet die Bilddaten zur weiteren Verarbeitung, Anzeige und Speicherung weiter an einen Computer 61. Ferner ist im Steuergerät oder im Computer 61 eine Möglichkeit zur Verknüpfung bzw. synoptischen Darstellung der verschiedenen Bilddaten vorgesehen, sowie ggf. die Erzeugung eines synthetischen Stereobilds.

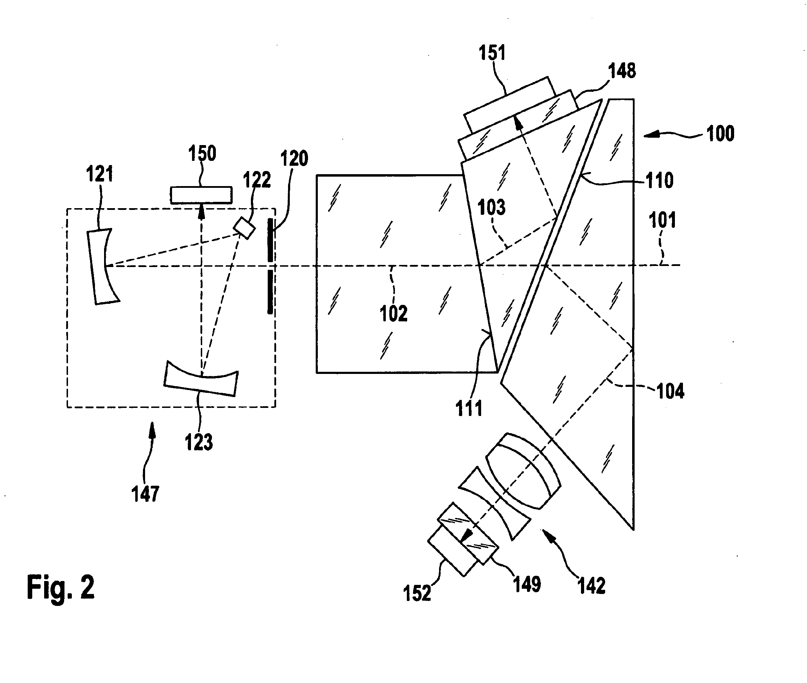

Eine alternative Ausgestaltung des Strahlteilers ist in Fig. 2 dargestellt. Anstelle des Strahlteilerwürfels 41 sind hier Prismen zur Aufteilung des Lichts vorgesehen, die einen Prismenblock 100 bilden.

Der einfallende Strahl 101 wird an zwei Grenzflächen 110 und 111 in insgesamt drei Kanäle 102, 103 und 104 aufgespalten. Diese Kanäle führen das Licht auf drei Bildsensoren 150, 151, 152, wovon mindestens einer ein phasenempfindlicher Bildsensor ist. Die einfallende Intensität kann bei metallischer Beschichtung farbneutral in vorbestimmten Intensitätsverhältnissen in die einzelnen Kanäle gespiegelt werden, bei dichroitischer Beschichtung auch farbselektiv oder spektral zerlegt. Durch Einführung zusätzlicher Flächen können auch mehr Kanäle erzeugt werden, durch ein einfaches Teilerprisma auch nur zwei Kanäle.

Vor dem Bildsensor 152 ist hier eine Anpassungsoptik 142 vorgesehen, sowie eine Aufbereitungsoptik 149, beispielsweise ein Filter. Auch die Aufbereitungsoptik vor dem Bildsensor 151 kann beispielsweise ein Filter 148 sein. Als Aufbereitungs- und Anpassungsoptik vor dem Bildsensor 150 dient in diesem Ausführungsbeispiel ein gekreuztes Czerny-Turner-Spektrometer 147. Durch die Linienblende 120 wird beispielsweise die mittlere Bildzeile selektiert und über den ersten Hohlspiegel 121, ein Beugungsgitter 122 oder auch ein diffraktives optisches Element oder ein Prisma und über den zweiten Hohlspiegel 123 auf den Bildsensor 150 abgebildet. Hierdurch kann ein spektral aufgelöstes und in einer Dimension räumlich aufgelöstes Bild erzeugt werden. Ein vollständiges Bild kann mosaikartig zusammengesetzt werden, indem die abgebildete Zeile über das Objekt geführt wird, beispielsweise durch Hin- und Herschwenken des Endoskops, was manuell oder motorisch durch eine entsprechende Schwenkeinrichtung beispielsweise bei einem Schwenkblick-Endoskop geschehen kann, oder durch automatisches Scannen durch Bewegung der Linienblende 120 und/oder des Spektrometers 147. Hierfür ist im Steuergerät eine entsprechende Steuerungs- und Auswertungssoftware installiert.

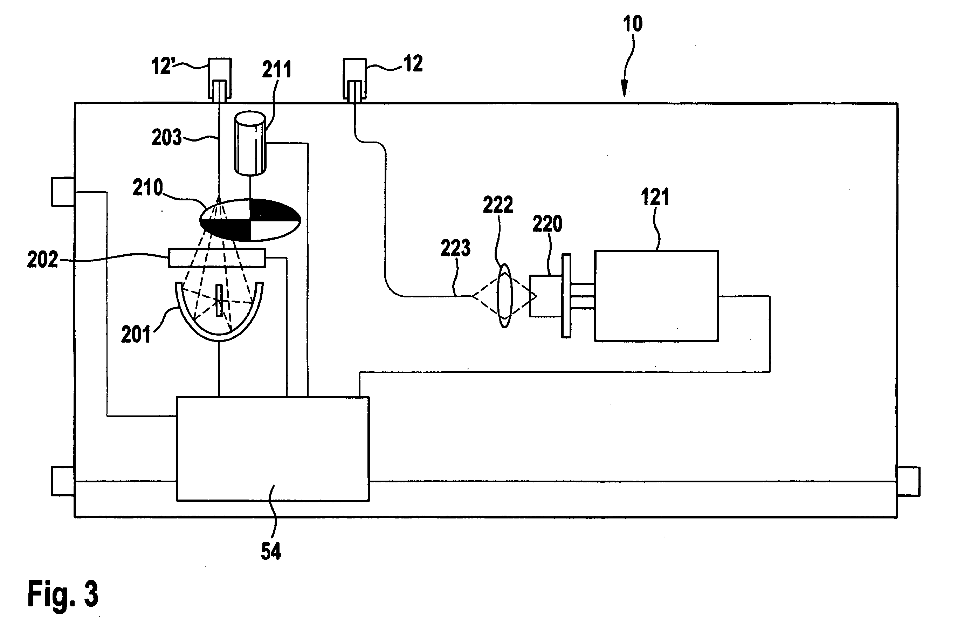

Wie in Fig. 3 dargestellt, kann erfindungsgemäß die Lichtquelle 10 das Steuergerät 54 enthalten, das mit der Steuerelektronik 53, Anzeigeeinrichtungen, wie beispielsweise einem Bildschirm 60, sowie weiteren Geräten, etwa einem Computer 61 kommuniziert und hierfür über Steckverbindungen verbindbar ist. Das Steuergerät steuert ferner die Lichterzeugung, beispielsweise von Weißlicht und der Fluoreszenzanregungsstrahlung.

Hierfür enthält die Lichtquelle 10 als Weißlichtquelle eine Metall-Halid-Bogen-Entladungslampe 201, die einen Reflektor umfassen kann, sowie weitere Elemente zur Kollimation bzw. Einkopplung in einen Lichtleiter 203. Alternativ können auch LED-, Xenon- oder Halogenlampen als Weißlichtquelle verwendet werden, ebenso wie RGB- oder Superkontinuum-Laserquellen. Um zu verhindern, dass das Weißlicht die Fluoreszenzmessung beeinträchtigt, ist ein Chopperrad 210 vorgesehen, das den Lichtfluss unterbricht, sobald eine Fluoreszenzmessung stattfindet. Dies kann manuell eingegeben werden, um abwechselnd im Weißlicht und im Fluoreszenzlicht zu beobachten, wobei ein Fluoreszenzlebensdauerbild (FLIM) oder ein Fluoreszenzintensitätsbild (AF/PDD) aufgenommen werden kann. Es kann aber auch automatisch, insbesondere im Videotakt oder einem Bruchteil davon, zwischen Weißlicht und Fluoreszenzbeobachtung umgeschaltet werden. Das Steuergerät steuert den Antrieb 211 des Chopperrads entsprechend den jeweiligen Anforderungen, beispielsweise synchron mit dem Auslesen des jeweiligen Bildsensors. Anstelle eines Chopperrads kann auch ein Schwingspiegel oder ein elektronisch gesteuertes Filter verwendet werden. Bei Verwendung von Festkörperlichtquellen, beispielsweise LED- oder Laserlichtquellen können diese direkt im entsprechenden Takt angesteuert werden. Der Lichtleiter 203 leitet das Licht über einen Anschluss 12' in ein Lichtleitkabel ein.

Zur Erzeugung der Fluoreszenzanregungsstrahlung ist eine Laserdiode 220 vorgesehen, die über eine Treiberelektronik 221 angesteuert wird und deren Licht über eine Kollimationsoptik 222 in einen Lichtleiter 223 eingekoppelt wird. Stattdessen kann auch eine fasergekoppelte Leuchtdiode oder eine Superlumineszenzdiode eingesetzt werden. Das erzeugte Licht wird über einen Anschluss 12 in ein Lichtleitkabel zur Weiterleitung in das Endoskop eingeleitet. Die Laserdiode wird durch das Steuergerät synchron zum Auslesen des phasenempfindlichen Bildsensors moduliert.

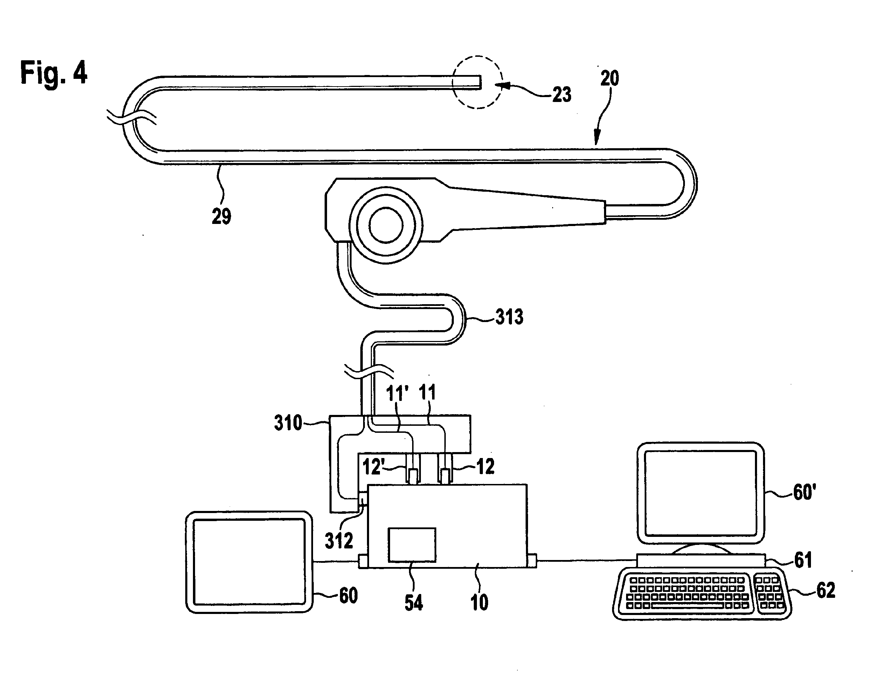

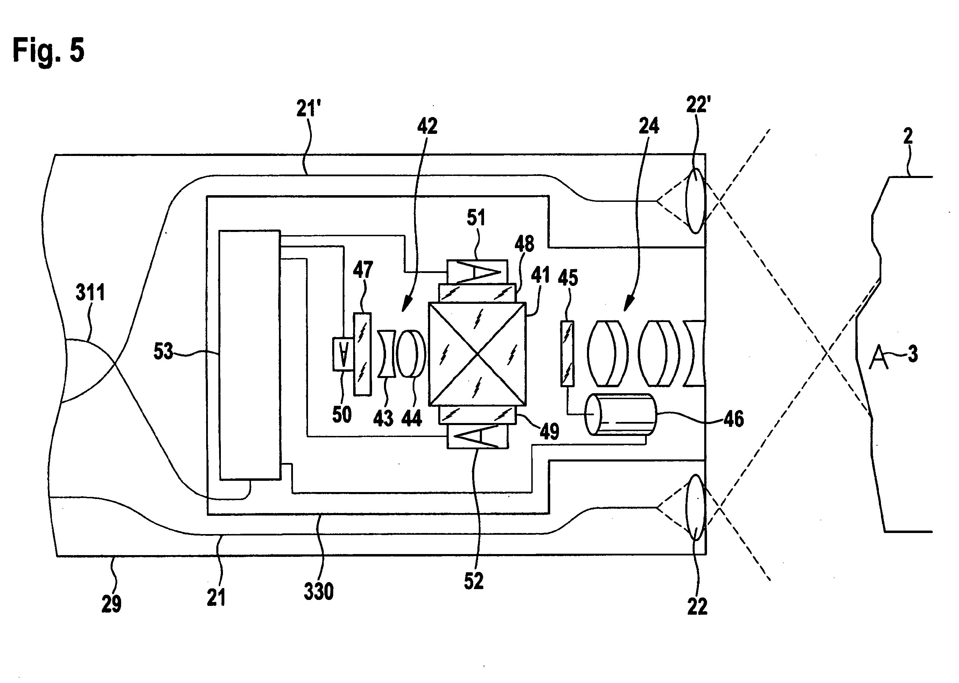

In der in Fig. 4 gezeigten Ausführungsform sind die optischen und elektronischen Bauelemente, die in dem in Fig. 1 dargestellten Ausführungsbeispiel der Kameraanordnung 30 zugeordnet sind, innerhalb des Endoskopschafts 29 nahe dessen distalem Ende 23 angeordnet, das in Fig. 5 im Detail dargestellt ist. Eine Relaislinsen-Optik bzw. ein Glasfaser-Bildleiter zur Weiterleitung des vom Endoskopobjektiv erzeugten Bilds an das proximale Ende des Endoskops sowie ein Okular sind hier nicht notwendig. Das Endoskopobjektiv 24 umfasst hier die Gesamtheit der Linsen vor dem Strahlteiler 41, ein zusätzliches Kameraobjektiv ist nicht notwendig. Die Aufbereitungsoptik 45, beispielsweise ein einschwenkbarer Filter, ist hier beispielsweise zwischen Endoskopobjektiv 24 und Strahlteiler 41 angeordnet. Das Endoskopobjektiv 24 und die der Kameraanordnung 30 zuzuordnenden Bauelemente können zu einer distalen Kameraeinheit 330 zusammengefasst sein, die über eine Kupplung (nicht dargestellt) lösbar mit dem verbleibenden Teil des Endoskops 20 verbunden sein kann und in der Art eines Wechselkopfs bzw. -objektivs gegen anderen Kameraeinheiten austauschbar sein kann. Die distale Kameraeinheit 330 kann auch die distale Beleuchtungsoptik, insbesondere die Aufweitungsoptiken 22, 22' und diesen zugeordnete Teile der Endoskop-Lichtleiter 21, 21', sowie das distale Ende des Endoskopschafts 29 umfassen.

Der Endoskopschaft 29 ist in diesem Ausführungsbeispiel flexibel, kann aber auch halbstarr oder starr sein. Die Steuerungselektronik 53 ist über ein Kabel 311, das gemeinsam mit den Lichtleitkabeln 11, 11' durch den Endoskopschaft 29 und ein Endoskopkabel 314 geführt ist, mit dem Steuergerät 54 verbunden. Zur Verbindung des Endoskops 20 mit der Lichtquelle 10 und mit dem Steuergerät 54 können die Anschlüsse 12, 12' und der Anschluss 312 des Kabels 54 in eine Anschlussbox 310 zusammengefasst sein.

Vorteilhafterweise entspricht der Aufbau des Endoskops 20 dem eines Standard-Videoskops. Zur Beschreibung weiterer Einzelheiten wird auf Fig. 1 verwiesen.

Soll ein Verfahren zur endoskopischen FLIM-Diagnose angewendet werden, so kann zuvor dem Patienten bzw. lokal dem zu beobachtenden Gewebebereich ein Photosensibilisator zugeführt werden, beispielsweise 5-Aminolävulinsäure, woraus durch Stoffwechselvorgänge die zur Fluoreszenz anregbaren Fluorophore entstehen. Bei einer Autofluoreszenz-Diagnose ist die vorherige Gabe von Photosensibilisatoren nicht erforderlich, allerdings ist das Fluoreszenzsignal dabei i. d. R. schwächer als bei durch einen Photosensibilisator induzierter Fluoreszenz. Je nach Körpergewebe und Zielsetzung der Diagnose, also beispielsweise ob degradierter Knorpel, Plaques, entartetes Gewebe oder auch anderes biologisches Material untersucht werden sollen, ist die Auto- oder die induzierte Fluoreszenz vorzuziehen, wobei auch eine Kombination beider Methoden möglich ist.

Sodann wird, ggf. nach entsprechender Vorbereitung des Patienten, das Endoskop 20 in die Körperhöhle, in der die Diagnose erfolgen soll, durch eine natürliche oder künstliche Körperöffnung eingeführt. Die Kameraanordnung 30 wird, sofern sie nicht eine Einheit mit dem Endoskop darstellt oder bereits mit diesem verbunden ist, an das Endoskop 20 angeschlossen, beispielsweise über die Endoskopkupplung 32 an die Okularmuschel 28 angeclippt. Sofern die Lichterzeugungsmittel nicht Teil des Endoskops sind, wird die Lichtquelle 10 mit dem Endoskop 20 über die Lichtkabel 11, 11' verbunden. Vorteilhafterweise wird das Endoskop 20 an einem Halter befestigt, der eine Bewegung des Endoskops relativ zum untersuchten Objekt während der Untersuchung verhindert.

Zur Durchführung des Verfahrens zur Fluoreszenz-Bildgebung wird in der Lichtquelle 10 das Beleuchtungslicht erzeugt, insbesondere die Fluoreszenzanregungsstrahlung und Weißlicht. Die Fluoreszenzanregungsstrahlung umfasst insbesondere Wellenlängen im kurzwelligen Teil des sichtbaren Spektrums oder im nahen UV, da Licht dieser Wellenlängen gut für die Anregung vieler Fluorophore geeignet ist. Insbesondere kann dies durch eine Superlumineszenzdiode oder einen Diodenlaser erzeugte Strahlung von 405 nm oder 445 nm sein. Das Weißlicht kann beispielsweise das ganze sichtbare Spektrum oder einen Teil davon umfassen, kann aber auch aus einem oder mehreren schmalbandigen Anteilen bestehen. Die Fluoreszenzanregungsstrahlung ist mit einer Frequenz von beispiels-weise ca. 10 - 100 MHz sinusförmig intensitätsmoduliert. Vorteilhafterweise ist das Weißlicht im Videotakt schaltbar.

Weißlicht und Fluoreszenzanregungsstrahlung werden über die Lichtkabel 11, 11' und die Lichtleiter 21, 21' auf das zu beobachtende Gebiet geleitet. Über die Abbildungsoptik wird auf dem phasenempfindlichen Bildsensor 50 ein Fluoreszenzbild erzeugt, wobei die Aufbereitungsoptik 47 vor dem Bildsensor 50 als Langpassfilter ausgebildet ist, das für die Fluoreszenzanregungsstrahlung vollständig oder nahezu vollständig undurchlässig ist. Durch mit der Modulation der Fluoreszenzanregungsstrahlung synchronisiertes Auslesen des Bildsensors werden pixelweise phasenabhängige Daten gewonnen, die von der Steuerelektronik 53 und dem Steuergerät 54 zu Intensitäts- und Phaseninformationen verarbeitet werden. Das Steuergerät erzeugt dadurch ein Fluoreszenzphasenbild, das die Zeitverzögerung der Fluoreszenzstrahlung gegenüber der Fluoreszenzanregungsstrahlung bzw. die Fluoreszenzlebensdauer darstellt. Ebenso kann ein Fluoreszenzintensitätsbild erzeugt werden.

Das FLIM- bzw. Fluoreszenzlebensdauerbild kann für den Arzt auf einem Bildschirm dargestellt werden und für weitere Bildverarbeitungsschritte oder für die Speicherung zur Verfügung stehen. Zur Verbesserung der Spezifität der Diagnose ist es vorteilhaft, auch das Fluoreszenzintensitätsbild oder auch ein RGB-Bild heranzuziehen, das zu diesem Zweck beispielsweise abwechselnd oder in Überlagerung mit dem Fluoreszenzlebensdauerbild dargestellt werden kann.

Mit dem phasenempfindlichen Bildsensor kann auch ein 3D-Bild des beobachteten Gebiets aufgenommen werden. Hierzu wird beispielsweise das Weißlicht in gleicher Weise wie die Fluoreszenzanregungsstrahlung moduliert, wobei zur Vermeidung der Fluoreszenzstrahlung fluoreszenzanregende Anteile noch in der Lichtquelle 10 herausgefiltert werden können. Es kann aber auch die Fluoreszenzanregungsstrahlung hierfür benutzt werden, wobei die Fluoreszenzstrahlung durch ein Kurzpassfilter vor dem Bildsensor 50 herausgefiltert werden kann. Hierfür können die Filter auch schwenkbar angeordnet sein. In der Regel wird aber die Fluoreszenzstrahlung so schwach sein, dass hierdurch kein wesentlicher Meßfehler entsteht. Das Auslesen der Pixel und die Auswertung der 3D-Daten erfolgt in gleicher Weise wie beim Fluoreszenzlebensdauerbild, mit dem Unterschied, dass hier alleine bzw. überwiegend reflektierte bzw. gestreute Strahlung aufgenommen wird, bei der die Zeitverzögerung die entfernungsbedingte Laufzeit des Lichts darstellt. Auf diese Weise können 3D-Daten erzeugt werden, die für den Beobachter ebenfalls beispielsweise auf einem Videoschirm dargestellt werden können.

## Patentansprüche

1. Vorrichtung zur Fluoreszenz-Bildgebung, mit:
- Lichterzeugungsmitteln zur Erzeugung mindestens einer modulierten Fluoreszenzanregungsstrahlung,
- Lichtweiterleitungsmitteln zur Weiterleitung der Fluoreszenzanregungsstrahlung auf einen zu beobachtenden Bereich, wobei die Lichtweiterleitungsmittel mindestens eine endoskopisch einsetzbare Beleuchtungsoptik umfassen,
- Lichtabbildungsmitteln zur Abbildung einer Fluoreszenzstrahlung aus dem zu beobachtenden Bereich mindestens auf einen ersten Bildsensor (50), der eine Mehrzahl von Pixeln aufweist, wobei die Lichtabbildungsmittel mindestens eine endoskopisch einsetzbare Abbildungsoptik umfassen, und
- Steuerungs- und Auswertungsmitteln zur Steuerung der Lichterzeugungsmittel, zur Ansteuerung des ersten Bildsensors und zur Auswertung der von dem ersten Bildsensor gelieferten Daten zur Erzeugung eines Fluoreszenzbilds, das ortsaufgelöste Fluoreszenzlebensdauerinformationen darstellt,
wobei
- die Fluoreszenzanregungsstrahlung kontinuierlich modulierbar ist,
- der erste Bildsensor (50) ein Festkörperdetektor ist, der phasenempfindlich ansteuerbar ist,
- die von dem ersten Bildsensor (50) gelieferten Daten pixelweise Phaseninformationen der Fluoreszenzstrahlung enthalten,
- mindestens ein weiterer Bildsensor (51, 52) zur Erzeugung eines Bilds mittels des reflektierten und/oder gestreuten Lichts vorgesehen ist,
- die Lichtabbildungsmittel einen Strahlteiler (41) zur Aufteilung der empfangenen Strahlung auf die Bildsensoren (50, 51, 52) umfassen,
- die Beleuchtungsoptik und die Abbildungsoptik in einem gemeinsamen endoskopisch einsetzbaren Schaft (29) angeordnet sind,
- eine Kameraanordnung (30) vorgesehen ist, die mindestens den ersten Bildsensor (50) enthält, und die mit dem endoskopisch einsetzbaren Schaft (29) lösbar verbunden ist und
- die Vorrichtung zur Erzeugung von 3D-Daten in Form von pixelweisen Abstandsinfolmationen mittels des ersten Bildsensors (50) durch Verwendung von reflektiertem bzw. gestreutem Licht ausgebildet ist.

2. Vorrichtung nach Anspruch 1, **dadurch gekennzeichnet, dass** die Beleuchtungsoptik, die Abbildungsoptik und der gezneinsame endoskopisch einsetzbare Schaft (29) Teile eines Standard-Endoskops (20) sind.

3. Vorrichtung nach Anspruch 1 oder 2, **dadurch gekennzeichnet, dass** Mittel zur Kohärenzreduktion vorgesehen sind.

4. Vorrichtung nach einem der Ansprüche 1 bis 3, **dadurch gekennzeichnet, dass** die Lichtabbildungsmittel einen optischen Umschalter umfassen.

5. Vorrichtung nach einem der Ansprüche 1 bis 4, **dadurch gekennzeichnet, dass** eine Anpassungsoptik (42) vorgesehen ist zur Anpassung der Gesichtsfelder des ersten Bildsensors (50) und mindestens eines weiteren Bildsensors (51, 52).

6. Vorrichtung nach einem der Ansprüche 1 bis 5, **dadurch gekennzeichnet, dass** das mittels des reflektierten und/oder gestreuten Lichts erzeugte Bild spektral aufgelöst bzw. selektiert ist.

7. Vorrichtung nach Anspruch 6, **dadurch gekennzeichnet, dass** das spektral selektierte Bild durch Punkt- oder Linienscannen gewonnen wird.

8. Vorrichtung nach einem der Ansprüche 1 bis 7, **dadurch gekennzeichnet, dass** ein Bildsensor (50, 51, 52) zur Erzeugung eines Fluoreszenzintensitätsbilds vorgesehen ist.

9. Vorrichtung nach einem der Ansprüche 1 bis 8, **dadurch gekennzeichnet, dass** die Steuerungs- und Auswertemittel zur Korrektur der Fluoreszenzlebensdauerdaten durch Verwendung der 3D-Daten ausgebildet sind.

## Claims

1. Device for fluorescence imaging, comprising:
- light-generation means for generating at least one modulated fluorescence excitation radiation,
- light-transmission means for transmitting the fluorescence excitation radiation to a region to be observed, wherein the light-transmission means comprise at least one illumination optical unit which can be used endoscopically,
- light-imaging means for imaging fluorescence radiation from the region to be observed, at least on a first image sensor (50), which comprises a plurality of pixels, wherein the light-imaging means comprise at least one imaging optical unit which can be used endoscopically, and
- control and evaluation means for controlling the light-generation means, for actuating the first image sensor and for evaluating the data supplied by the first image sensor for generating a fluorescence image, which represents spatially resolved fluorescence lifetime information,
wherein
- the fluorescence excitation radiation can be modulated continuously,
- the first image sensor (50) is a solid-state detector, which can be actuated in a phase-sensitive manner,
- the data supplied by the first image sensor (50) contain pixel-by-pixel phase information of the fluorescence radiation,
- at least one further image sensor (51, 52) is provided for generating an image by means of the reflected and/or scattered light,
- the light imaging means comprise a beam splitter (41) for splitting the received radiation onto the image sensors (50, 51, 52),
- the illumination optical unit and the imaging optical unit are arranged in a common shaft (29) which can be used endoscopically,
- a camera arrangement (30) is provided, which contains at least the first image sensor (50) and which is detachably connected to the shaft (29) which can be used endoscopically, and
- the device is embodied for generating 3D data in the form of pixel-by-pixel distance information by means of the first image sensor (50) by using reflected and/or scattered light.

2. Device according to Claim 1, **characterized in that** the illumination optical unit, the imaging optical unit and the common shaft (29) which can be used endoscopically are parts of a standard endoscope (20).

3. Device according to Claim 1 or 2, **characterized in that** means for coherence reduction are provided.

4. Device according to one of Claims 1 to 3, **characterized in that** the light-imaging means comprise an optical switching means.

5. Device according to one of Claims 1 to 4, **characterized in that** an adaptation optical unit (42) is provided for adapting the fields of view of the first image sensor (50) and of at least another image sensor (51, 52).

6. Device according to one of Claims 1 to 5, **characterized in that** the image generated by means of the reflected and/or scattered light is spectrally resolved or selected.

7. Device according to Claim 6, **characterized in that** the spectrally selected image is obtained by point scanning or line scanning.

8. Device according to one of Claims 1 to 7, **characterized in that** an image sensor (50, 51, 52) for generating a fluorescence intensity image is provided.

9. Device according to one of Claims 1 to 8, **characterized in that** the control and evaluation means are embodied for correcting the fluorescence lifetime data by using the 3D data.

## Revendications

1. Dispositif d'imagerie à fluorescence comprenant :
- des moyens générateurs de lumière destinés à générer au moins un rayonnement d'excitation de fluorescence modulé,
- des moyens de retransmission de lumière destinés à retransmettre le rayonnement de mesure sur une zone à observer, les moyens de retransmission de lumière comprenant au moins une optique d'éclairage utilisable de manière endoscopique,
- des moyens de formation d'image de lumière destinés à former l'image d'un rayonnement de fluorescence provenant de la zone à observer sur au moins un premier capteur d'image (50) présentant une pluralité de pixels, les moyens de formation d'image de lumière comprenant au moins une optique de formation d'image utilisable de manière endoscopique, et
- des moyens de commande et d'évaluation destinés à commander le moyen générateur de lumière pour commander le premier capteur d'image et évaluer les données délivrées par le premier capteur d'image afin de générer une image de fluorescence,
qui représente des informations de durée de vie de fluorescence à résolution spatiale,
- le rayonnement d'excitation de fluorescence pouvant être modulé en continu,
- le premier capteur d'image (50) étant un détecteur solide qui peut être commandé de manière sensible à la phase,
- les données fournies par le premier capteur d'image (50) contenant des informations de phase, mises en oeuvre sous forme de pixels, du rayonnement de fluorescence,
- au moins un capteur d'image supplémentaire (51, 52) étant prévu pour générer une image au moyen de la lumière réfléchie et/ou diffusée,
- les moyens de formation d'image de lumière comprenant un diviseur de faisceaux (41) destiné à diviser le rayonnement reçu sur les capteurs d'image (50, 51, 52),
- l'optique d'éclairage et l'optique de formation d'image étant disposées dans une tige commune utilisable de manière endoscopique (29),
- l'optique d'éclairage et l'optique de formation d'image sont disposées dans une tige commune utilisable de manière endoscopique (29),
- un système de caméra (30) étant prévu, comportant au moins le premier capteur d'image (50) et connecté de manière amovible à la tige utilisable de manière endoscopique (29), et
- le dispositif de génération de données 3D sous forme d'informations de distance mises en oeuvre sous forme de pixels étant réalisé au moyen du premier capteur d'image (50) en utilisant la lumière réfléchie ou diffusée.

2. Dispositif selon la revendication 1, **caractérisé en ce que** l'optique d'éclairage, l'optique de formation d'image et la tige commune utilisable de manière endoscopique (29) font partie d'un endoscope standard (20).

3. Dispositif selon la revendication 1 ou 2, **caractérisé en ce qu'**il est prévu des moyens destinés à réduire la cohérence.

4. Dispositif selon l'une quelconque des revendications 1 à 3, **caractérisé en ce que** les moyens de formation d'image de lumière comprennent un dispositif de permutation optique.

5. Dispositif selon l'une quelconque des revendications 1 à 3, **caractérisé en ce qu'**il est prévu au moins une optique d'adaptation (42) destinée à adapter le champ visuel au premier capteur d'image (50) et d'au moins un autre capteur d'image (51, 52).

6. Dispositif selon l'une quelconque des revendications 1 à 5, **caractérisé en ce que** l'image générée au moyen de la lumière réfléchie et/ou diffusée est résolue ou sélectionnée spectralement.

7. Dispositif selon la revendication 6, **caractérisé en ce que** l'image sélectionnée spectralement est obtenue par balayage ponctuel ou linéaire.

8. Dispositif selon l'une quelconque des revendications 1 à 7, **caractérisé en ce qu'**il est prévu un capteur d'image (50, 51, 52) destiné à générer une image d'intensité de fluorescence.

9. Dispositif selon l'une quelconque des revendications 1 à 8, **caractérisé en ce que** les moyens de commande et d'analyse sont réalisés pour la correction des données de durée de vie de fluorescence en utilisant les données 3D.
